# EUROPEAN PATENT APPLICATION

(11) **EP 4 763 180 A1**
(43) Date of publication of application: **24.06.2026**
(21) Application number: 25218934.5
(22) Date of filing: 27.11.2025
(51) Int. Cl.: A61J 1/06, A61J 1/14, A61M 5/142

(54) **DISPENSING ASSEMBLY FOR DISPENSING A MEDICAMENT**

(30) Priority: 20.12.2024 IT 202400029532
(71) Applicant: Nuova Ompi S.r.l., 35017 Piombino Dese, (PD) (IT)
(72) Inventor: RIVA, CHRISTIAN, I-20092 Cinisello Balsamo (MI) (IT); CATTANEO, MATTIA, I-20816 Ceriano Laghetto (MB) (IT); CUCCATO, ANDREA, I-20131 Milano (MI) (IT); DEGAN, PAOLO, I-24020 Ranica (BG) (IT); BOKELMAN, KEVIN LEE, San Diego, CA, 92130 (US); LIVINGSTON, ADAM JOSEPH, Oceanside, CA, 92056 (US); ENGELMANN, MICHAEL GEORGE, San Diego, CA, 92131 (US); CARLSON, ANDREW JAMES, Hopkins, MN, 55343 (US); UBACH, ANTONIO JOAO, Tucson, AZ, 85711 (US)
(74) Representative: Porta & Consulenti Associati S.p.A.

(57) **Abstract**

Dispensing assembly (10) for dispensing a medicament, comprising a cartridge (20) and a needle supporting member (42). The cartridge (20) comprises a perforable septum (24) and a first aseptic chamber (25) defined between the perforable septum (24) and a first perforable membrane (27) arranged at an end of the cartridge (20) proximal to the needle supporting member (42). The latter comprises a perforating needle (32) and a second aseptic chamber (45) defined around the perforating needle (32) between a closed wall (44) which is distal from the cartridge (20) and a second perforable membrane (47) associated with the needle supporting member (42) at an end thereof proximal to the cartridge (20). The dispensing assembly (10) is configured to pass from a first operating configuration in which the perforating needle (32) is distant from the perforable septum (24) and a second operating configuration in which the perforating needle (32) has perforated the perforable septum (24). During this passage, the second perforable membrane (47) is initially perforated by a first perforating member (28) associated with the cartridge (20) and the first perforable membrane (27) is perforated by a second perforating member (48) associated with the needle supporting member (42) and, subsequently, the perforating needle (32) perforates the perforable septum (24).

## Description

The present invention relates to a dispensing assembly for dispensing a medicament.

The invention also relates to a method for making an aseptic connection between a cartridge containing a medicament to be dispensed and a perforating needle. This method can be implemented using the aforementioned dispensing assembly.

Preferably, the dispensing assembly of the invention is used in a medicament dispensing device, more preferably in a subcutaneous medicament dispensing device.

Therefore, the invention also relates to such a device comprising the aforementioned dispensing assembly.

The dispensing device of the present invention falls within the category of the "infusion pumps" as defined by the FDA (US Food and Drug Administration) and relates, more preferably, to a wearable type device, i.e. a device configured to be applied on the body of a patient in order to allow the subcutaneous administration of a predetermined dose of the medicament. The application of the device on the body of the patient is carried out by a user, for example a doctor or a nurse or the patient.

An example of a dispensing device of the type discussed above is described in WO 2020/128821A1.

In all the subcutaneous dispensing devices the medicament is initially contained in a cartridge housed within the device and is transferred to the body of the patient via a fluidic path.

In the present description and the subsequent claims, the expression "fluidic path" is used to refer to any element or assembly of elements that is configured to be connected to a cartridge and to allow the passage of the medicament from the cartridge to the patient's body.

To this end, the fluidic path comprises, at a first end thereof, a perforating needle configured to be inserted into the cartridge, at a second end thereof opposite to the aforementioned first end, an injection needle intended to be inserted into the patient's body and, between the aforementioned first and second ends, a flexible tube which put the perforating needle in fluid communication with the injection needle.

Typically, the cartridge comprises a cylindrical container, usually made of plastic or glass material, a plunger slidable inside the container to push the medicament out of the container and a perforable septum.

The container of the cartridge is kept closed by the perforable septum until the administration of the medicament is required. When such an administration is required, the perforating needle perforates the perforable septum and opens the fluidic path, allowing the medicament to reach the patient by initially passing through the perforating needle, then through the flexible tube and finally through the injection needle, due to the thrust exerted by the plunger on the medicament inside the container.

For the safety of the patient, it is essential to ensure the sterility of the fluidic path and, consequently, of the outer interface surfaces between the cartridge and the fluidic path (i.e. the outer surface of the perforating needle and the outer surface of the perforable septum that will be perforated by the perforating needle) and the outer surface of the injection needle.

Some devices require the user, when using the device, to insert the cartridge into the device and connect it to the fluidic path. In such devices, the user should clean the aforementioned outer surfaces to remove any hazardous micro-organisms.

The Applicant believes that such devices have the drawback of having to carry out a plurality of operations which, in addition to being time-consuming, require a prior training of the user, this being absolutely necessary to ensure a correct installation of the cartridge and to achieve the desired sterility.

In other devices, insertion of the cartridge takes place during the assembly of the device.

In this case, a terminal sterilisation of the already assembled device or the assembly of the entire medicament dispensing device in an aseptic environment may be foreseen to achieve the desired sterility.

However, even these solutions have their drawbacks. In fact, terminal sterilisation cannot be carried out when dealing with certain categories of medicaments (e.g. in the case of complex drug formulations such as monoclonal "MABs" or biologics antibodies), while the assembly of the entire device in an aseptic environment entails high costs for creating and maintaining such an environment and a complexity in the management of workflows and materials in the aseptic environment, with a consequent increase in the time required to complete the assembly.

Devices are known in which the sterility of the outer interface surfaces between the cartridge and the perforating needle is achieved by arranging these surfaces in respective aseptic chambers closed by respective membranes.

For example, in US 11752258B2 membranes intended to be removed before the device is used are provided. In particular, in a solution described and shown, a first membrane is arranged on the outer surface of the perforable septum and a second membrane is placed on a wall of the member that supports the perforating needle.

In US 10569014B2, membranes intended to be perforated prior to the start of the medicament administration are provided. In particular, a first perforable membrane closes an aseptic chamber defined in the perforable septum and a second perforable membrane closes an aseptic chamber defined around the perforating needle in the member that supports the perforating needle. The two perforable membranes are arranged one immediately after the other so that they can be perforated almost simultaneously by a perforating element provided on the member that supports the perforating needle.

The Applicant believes that the use of membranes at the perforable septum and the perforating needle allows the cartridge and the member that supports the perforating needle to be assembled individually and separately in aseptic environment and thus to maintain the sterility of the perforable septum and the perforating needle until just moments before the administration of the medicament. Since it is not necessary to assemble the entire medicament dispensing device in an aseptic environment, the management of workflows and materials in the aseptic environment is greatly simplified.

The Applicant therefore focused on solutions in which membranes are provided at the perforable septum and the perforating needle.

The Applicant observed that all dispensing devices must be designed taking in due consideration the forces generated in the device once the device is activated, primarily the force required to allow the perforation of the perforable septum by the perforating needle and the reaction force generated in the device at the moment the perforable septum is perforated by the perforating needle.

The Applicant also observed that in order to achieve an advantageous design repeatability, it is desirable to be able to predict as accurately as possible the magnitude of all forces that are generated in the device once the device is activated and that, to this end, it would be desirable for only axial forces to be present in the device, i.e., only forces oriented along a direction parallel to or coinciding with the direction of movement of the perforating needle.

In this description and the appended claims, the terms "axial" and the like are used to refer to any direction coincident with or parallel to the direction along which the perforating needle and/or cartridge extends, the terms "radial" and the like are used to refer to any direction perpendicular to the axial direction, and the terms "transverse" and the like are used to refer to any non-axial direction.

The Applicant believes that in devices in which removable membranes are used, such as in the devices described in US 11752258B2, removal of the membranes results in high transverse forces being applied to the devices at the interface between the cartridge and the member that supports the perforating needle.

For this reason, the Applicant focused on solutions in which perforable membranes are used. In fact, the Applicant believes that even if perforable membranes generate reaction forces that inevitably have undesirable transverse components, the magnitude of these transverse components is in any case less than the transverse forces to be applied for the removal of removable membranes. Moreover, the perforation of the perforable membranes does not require any specific action to be carried out by the user, as it occurs automatically once the device is activated for administrating the medicament.

The Applicant observed that in the devices described in US 10569014B2 the two perforable membranes are substantially in contact with each other. Consequently, the perforating element must be able to perforate the two membranes almost simultaneously, and in order to do so, it must apply a high axial force to the membranes. These membranes in turn react, generating a high reaction force and, inevitably, high transverse force components in the device. The latter, although lower than the transverse stresses generated in devices provided with removable membranes, are nonetheless of such a magnitude as to be undesirable for the reasons explained above. In addition, the edges of the membrane that is perforated first interfere with the other membrane during the perforation of both membranes, with the risk of compromising the quality of the perforation, i.e. of achieving a non-optimal perforation.

The technical problem solved by the present invention is to provide a dispensing assembly for dispensing a medicament comprising perforable membranes that does not have the drawbacks discussed above.

The present invention therefore relates, in a first aspect thereof, to a dispensing assembly for dispensing a medicament, comprising:
- a cartridge comprising a container containing the medicament to be dispensed and a perforable septum coupled to the container at a dispensing end portion of the container;
- a perforating needle configured to enter the container at said dispensing end portion by perforating the perforable septum;
- an injection needle configured to inject the medicament to a patient, said injection needle defining with the perforating needle a fluidic path configured to be travelled by the medicament during dispensing of the medicament;
- a needle supporting member on which the perforating needle is mounted;
- a first aseptic chamber defined between the perforable septum and a first perforable membrane associated with the container at one end thereof proximal to the needle supporting member;
- a second aseptic chamber defined in the needle supporting member around the perforating needle between a closed wall which is distal from the cartridge and a second perforable membrane associated with the needle supporting member at one end thereof proximal to the cartridge;
wherein the dispensing assembly is configured to pass from a first operating configuration in which the perforating needle is distant from the perforable septum and the fluidic path is closed and a second operating configuration in which the perforating needle has perforated the perforable septum and the fluidic path is open;
wherein during a first part of the passage from said first operating configuration to said second operating configuration and before the perforating needle has perforated the perforable septum, said second perforable membrane is perforated by a first perforating member associated with the cartridge and the first perforable membrane is perforated by a second perforating member associated with the needle supporting member, and during a second part of the passage from said first operating configuration to said second operating configuration and after said first perforable membrane and second perforable membrane have been perforated, the perforating needle perforates the perforable septum.

The Applicant believes that due to the provision of two separate perforating members, each configured to perforate a respective perforable membrane, the axial force required by each perforating member to perforate the respective perforable membrane may have a magnitude less than that required in the devices described in US 10569014B2. Consequently, each of the two membranes generates a reaction force having small transverse components, to the benefit of the desired design repeatability. Furthermore, the provision of the two perforating members on the cartridge and on the needle supporting member, respectively, ensures that the edges of a newly perforated membrane do not interfere with the other membrane, resulting in an optimal perforation of both membranes without the risk of contaminating the perforable septum and the perforating needle before they come into contact with each other.

In a second aspect thereof, the present invention relates to a subcutaneous dispensing device for dispensing a medicament, comprising a dispensing assembly according to the first aspect of the present invention.

Such a device thus has all the advantages discussed here with reference to the dispensing assembly of the first aspect of the invention. The device also defines an essentially closed system, all the components necessary to enable its operation and the administration of the medicament being housed inside the device prior to its use.

In a third aspect thereof, the present invention relates to a method for creating an aseptic connection between a cartridge containing a medicament to be dispensed and a perforating needle, wherein the cartridge comprises a container containing the medicament to be dispensed, a perforable septum coupled to the container and a first aseptic chamber defined between the perforable septum and a first perforable membrane associated with the container at an end thereof proximal to the perforating needle, and wherein the perforating needle is arranged within a second aseptic chamber defined in a needle supporting member between a closed wall distal from the cartridge and a second perforable membrane associated with the needle supporting member at an end thereof proximal to the cartridge, the method comprising:
- perforating the second perforable membrane by a first perforating member associated with the cartridge and the first perforable membrane by a second perforating member associated with the needle supporting member and, subsequently,
- perforating the perforable septum by the perforating needle.

This method can be carried out by using the dispensing assembly according to the first aspect of the invention and thus has all the advantages discussed herein with reference to said dispensing assembly.

In a fourth aspect thereof, the present invention relates to a cartridge for a medicament dispensing device, comprising:
- a container containing the medicament to be dispensed;
- a perforable septum coupled to the container at a dispensing end portion of the container;
- a bushing coupled to the perforable septum and comprising:
   - a first annular wall proximal to the container and comprising a first opening closed by the perforable septum;
   - a second annular wall distal from the container and comprising a second opening closed by a perforable membrane;
   - a side wall joining the first annular wall to the second annular wall;
- an aseptic chamber defined within the bushing;
- a crimping ring coupled to the dispensing end portion of the container and in abutment against the bushing.

The aforementioned bushing coincides with what is referred to as the "first bushing" of the dispensing assembly in the remainder of this description and in the appended claims.

Such a cartridge may be used in the dispensing assembly of the first aspect of the invention and/or in the dispensing device of the second aspect of the invention.

The aspects of the invention discussed above allow the sterility of the perforable septum and the perforating needle to be maintained until just moments before the administration of the medicament.

As already mentioned, it is also crucial for the patient safety to ensure that the outer surface of the injection needle remains sterile.

It is also necessary to ensure an easy removal of the cap of the needle in order to be able to proceed with the administration of the medicament.

Devices are known in which the sterility of the injection needle is maintained by placing a cap over it.

For example, EP 3708202A1 describes a cap of an injection needle of a subcutaneous dispensing device. This cap comprises a rigid outer element that surrounds a sterile rubber core. The latter maintains the sterility of the needle while the rigid outer element protects the user from the risk of pricking. It is basically a standard cap of the so-called RNS (rigid needle shield) type.

Alternatively, the cap can consist of a single element, either in rubber or rigid.

EP 3708202A1 also describes a cap removal member. This removal member consists of two distinct elements, a cap hooking element and a user gripping element.

US 9427529B2 describes a removal member configured to remove a cap similar to that of EP 3708202A1.

The Applicant has thought to make a cap and a cap removal member different from those described in EP 3708202A1 and US 9427529B2 and in any case suitable for maintaining the sterility of the injection needle until a few moments before the administration of the medicament and to ensure easy removal of the cap just before proceeding with the administration of the medicament.

The present invention therefore relates, in a further aspect thereof, to a cap for an injection needle of a medicament dispensing device, comprising:
- a first cylindrical element made of a soft plastic material and configured to be coupled to an injection needle supporting member;
- a second cylindrical element made of a rigid plastic material; wherein the second cylindrical element comprises a first end portion coupled to the first cylindrical element and a second end portion having a closed end wall.

The element made of a soft plastic material ensures sterility through its compression action around the portion of the injection needle supporting member to which this element is coupled. In particular, unlike the cap described in EP 3708202A1 in which the rubber core is surrounded by a rigid outer element that inevitably hinders deformation of the rubber core, in the cap of the invention the soft plastic element is free to deform and can therefore adapt to the dimensional tolerances of coupling with the supporting member, sealing the environment around the injection needle under all potential conditions.

The rigid plastic element, on the other hand, ensures a good fit between the cap and the cap removal member.

In another aspect thereof, the present invention relates to a removal member for removing a cap of an injection needle of a medicament dispensing device, comprising a gripping portion and a coupling portion configured to be coupled to the cap, wherein the gripping portion and the coupling portion are made in a single piece.

Making the removal member in a single piece allows advantageous production cost and time savings. In particular, no assembly of the removal member is required before it is applied to the cap.

Preferred features of the invention are described below, each of which can be provided individually or in combination with the other features.

Preferably, the closed wall of the needle supporting member that is distal from the cartridge comprises an opening closed by an additional membrane. This membrane allows to keep aseptic the second aseptic chamber of the needle supporting member in which the perforating needle is arranged. This additional membrane may or may not be identical to the second perforable membrane.

Preferably, when the dispensing assembly is in the second operating configuration, the needle supporting member is arranged in a radially outer position with respect to the perforable septum. An advantageous optimisation of the space inside the dispensing device is thus achieved.

Preferably, during the first part of the passage from said first operating configuration to said second operating configuration the second perforable membrane is perforated by the first perforating member before the first perforable membrane is perforated by the second perforating member. Therefore, the two membranes are perforated at two separate times by non-simultaneous movement of the respective perforating members. There is thus a total physical and temporal separation of the perforation activities of the two membranes, which allows the perforable septum to be maintained in an aseptic environment even after the second perforable membrane provided on the needle supporting member has been perforated and the perforating needle to perforate the perforable septum without any risk of contamination before it reaches the perforable septum. This physical and temporal separation also helps to eliminate the risk of interference between the edges of one membrane just perforated and those of the other membrane to be perforated.

Preferably, the passage from said first operating configuration to said second operating configuration occurs as a result of a translational movement of the needle supporting member towards the cartridge.

In alternative embodiments, the passage from said first operating configuration to said second operating configuration occurs as a result of a translational movement of the cartridge towards the needle supporting member.

Preferably, in said first part of the passage from said first operating configuration to said second operating configuration, the needle supporting member has an initial configuration in which the second perforating member shifts towards the cartridge integrally with the needle supporting member. In this first part of the movement, the perforating needle remains inside the second aseptic chamber provided on the needle supporting member.

Preferably, in said second part of the passage from said first operating configuration to said second operating configuration, the needle supporting member has a final configuration in which the second perforating member is in abutment against the cartridge and the needle supporting member moves towards the cartridge. In this second part of the movement, the perforating needle is thus decoupled from the second perforating member to perforate the perforable septum.

Preferably, the first aseptic chamber is defined within a first bushing coupled to the perforable septum and having:
- a first annular wall in abutment against the perforable septum and comprising a first opening closed by the perforable septum;
- a second annular wall distal from the perforable septum and comprising a second opening closed by the first perforable membrane;
- a side wall joining the first annular wall to the second annular wall.

The first aseptic chamber is thus defined in a bushing arranged outside the container. This makes it possible to use a completely conventional container, which can thus be filled and moved by conventional machinery. Despite the provision of an aseptic chamber outside the container, the axial dimensions of this aseptic chamber are small, so that the cartridge can be housed inside devices made from conventional components without having to provide for additional space in order to accommodate the aforementioned aseptic chamber.

Preferably, the first bushing is held in abutment against the perforable septum by a crimping ring coupled to the dispensing end portion of the container. Such a crimping ring can also be a conventional component, and its application on the cartridge can occur in a conventional way.

Preferably, the first perforating member is defined by said second annular wall.

Preferably, the needle supporting member comprises a second bushing having:
- an annular wall proximal to the cartridge and comprising a respective opening closed by the second perforable membrane;
- said closed wall distal from the cartridge;
- a side wall joining the annular wall proximal to the cartridge to the closed wall distal from the cartridge.

Preferably, the second aseptic chamber is defined within the second bushing.

Preferably, the second perforating member is arranged in said second aseptic chamber around the perforating needle.

Preferably, the second perforating member comprises a central annular element surrounding the perforating needle.

Preferably, such a central annular element has one end proximal to the cartridge and configured to perforate the first perforable membrane during said first part of the passage from said first operating configuration to said second operating configuration.

More preferably, said central annular element is arranged coaxially to the perforating needle, in a radially outer position with respect to the perforating needle. The aforementioned central annular element thus serves as a protective element for the perforating needle both before and after the perforation of the second perforable membrane.

Preferably, when the dispensing assembly is in its first operating configuration the second annular wall of the first bushing is arranged at a first distance from the second perforable membrane and the end proximal to the cartridge of said central annular element is arranged at a second distance from the first perforable membrane, wherein the first distance is less than the second distance. This provision allows achieving at first the perforation of the second perforable membrane and only later that of the first perforable membrane.

Preferably, the second bushing comprises, on a radially inner surface thereof, at least one first sliding element.

Preferably, the second bushing comprises, on a radially inner surface thereof, at least one first locking element.

Preferably, the second perforating member comprises at least one second sliding element coupled to the at least one first sliding element.

Preferably, the second perforating member comprises at least one second locking element configured to be coupled to the at least one first locking element when the dispensing assembly is in its first operating configuration.

Preferably, said at least one second locking element is configured to decoupled from the at least one first locking element after said first perforable membrane and second perforable membrane have been perforated and after the second perforating member is in abutment against the cartridge.

The locking elements described above allow for the simultaneous movement (in the first part of the passage from the first operating configuration to the second operating configuration) of the second bushing and the second perforating member to achieve the perforation of the second perforable membrane and the first perforable membrane and the passage of the dispensing assembly from its first operating configuration to its second operating configuration after the perforation of the aforementioned perforable membranes and before the perforating needle perforates the perforable septum.

On the other hand, the sliding elements described above allow for the movement (in the second part of the passage from the first to the second operating configuration) of the second bushing with respect to the second perforating member to allow the perforating needle to perforate the perforable septum.

Preferably, the second perforating member is made of a material such that, following the abutment between the second perforating member and the cartridge, the second perforating member is deformed so as to decouple the at least one second locking element from said at least one first locking element. The passage of the dispensing assembly from its first operating configuration to its second operating configuration therefore occurs by deformation of the second perforating member when the latter is in abutment against the cartridge and continues to be pushed towards the cartridge.

Preferably, the second perforable membrane comprises a plurality of weakening lines. This provision makes it possible to limit the force required to achieve the perforation of the second perforable membrane, in addition to reproducibly predict the magnitude of this force.

Preferably, the weakening lines are arranged in a radial pattern. This provision allows a precise and predictable (and therefore reproducible) rupture of the second perforable membrane to be achieved, without the risk of creating unwanted membrane fragments having a shape and size that cannot be foreseen beforehand.

In some embodiments, the first membrane also comprises a plurality of weakening lines, preferably arranged in a radial pattern.

Preferably, the second perforable membrane is perforated by the first perforating member before the first perforable membrane is perforated by the second perforating member.

Preferably, the perforation of the first perforable membrane and of the second perforable membrane occurs as a result of a translational movement of the needle supporting member towards the cartridge.

Preferably, the perforation of the perforable septum occurs after the second perforating member has abutted against the cartridge as a result of a translational movement of the needle supporting member towards the cartridge and with respect to the second perforating member.

Preferably, the first annular wall is in direct abutment against the perforable septum.

Preferably, the crimping ring is in direct abutment against the bushing (corresponding to the aforementioned first bushing) and holds the first annular wall in abutment against the perforable septum.

In this description and subsequent claims, the term "direct abutment" is used to indicate an abutment between two elements without interposition of further elements.

Preferably, the first annular wall comprises an outer annular edge protruding towards the perforable septum. This outer annular edge presses against the perforable septum at an outer annular edge of the latter, ensuring a seal between the bushing and the perforable septum.

Preferably, the bushing comprises an annular flange having an increased outer diameter and comprising said first annular wall.

Preferably, the bushing comprises an annular portion having a reduced outer diameter, protruding from said annular flange and comprising said second annular wall.

Preferably, the crimping ring is fitted onto said annular portion.

Preferably, the crimping ring is in abutment against said annular flange.

Preferably, said annular portion comprises, on one a side surface thereof, at least one overhanging hooking element.

Preferably, the crimping ring comprises at least one locking element.

The aforementioned hooking element and locking element are configured to come into contact with each other and prevent the crimping ring from slipping out of the bushing once these components have been associated with each other. In this way, these components can be handled as if they were a single component during transport and assembly operations.

Preferably, the crimping ring comprises a main annular portion having a first diameter and a top portion provided with a through opening having a second diameter less than the first diameter.

Preferably, said at least one locking element is defined by a top surface of an edge delimiting said through opening. The locking element is thus formed at the aforementioned edge by deformation of the latter as a result of the insertion of the annular portion with a reduced outer diameter of the bushing into the through opening when the hooking element comes into contact with this edge.

Preferably, the container comprises a main body and, at said dispensing end portion, a neck.

Preferably, the perforated septum comprises a first portion inserted into the neck and a second portion arranged outside the container.

Preferably, said second portion comprises a first surface in abutment against a free end of the neck and a second surface in abutment against the first annular wall of the bushing.

Any conventional perforable septum of similar shape can therefore be used as the perforable septum.

Preferably, the first cylindrical element of the cap is co-molded to the second cylindrical element of the cap.

Preferably, the second cylindrical element comprises, on a side surface thereof, a recess. This recess allows the cap to remain in position with respect to the dispensing device during the coupling between the cap and the removal member, avoiding unwanted contact between the cap and the injection needle during the aforementioned coupling.

Preferably, the second cylindrical element comprises an end portion comprising a lateral surface having a conical surface portion near the recess. This surface portion is laterally abutted against a corresponding surface portion provided in the removal member when the latter is coupled to the cap.

Preferably, the aforementioned end portion comprises a cylindrical surface portion adjacent to the conical surface portion. This surface portion laterally abuts against a corresponding surface portion provided in the removal member when the latter is coupled to the cap.

Preferably, the aforementioned end portion comprises an undercut surface portion adjacent to the cylindrical surface portion. This surface portion abuts against a corresponding surface portion provided in the removal member when the latter is coupled to the cap and prevents decoupling between the cap and the removal member.

The above-described surface portions of the cap allow for a snap-fit coupling between the cap and the removal member, ensuring the mutual centring of these components and preventing them from a subsequent decoupling.

Preferably, the gripping portion of the removal member comprises two parts extending from opposite sides with respect to the coupling portion.

Preferably, the removal member comprises an opening interposed between each of said two parts and the coupling portion.

Preferably, the coupling portion comprises at least two guide elements facing each other. These guide elements ensure that the coupling portion of the removal member is centred with respect to the cap when the latter is coupled to the removal member.

Preferably, the coupling portion comprises at least two hooking elements facing each other. These hooking elements abut against the cap when the removal member is coupled to the cap.

Preferably, the coupling portion is elastically deformable. This elastic deformation is achieved by the provision of the aforementioned openings in the removal member and allows for a snap-fit coupling between removal member and cap.

Preferably, said at least two guide elements are aligned along a first direction and said at least two hooking elements are aligned along a second direction orthogonal to said first direction. This provision allows a precise centring and coupling between the removal member and the cap.

Preferably, each of said at least two hooking elements comprises a side surface having a conical surface portion. The conical surface portions of the removal member laterally abut against the conical surface portion of the cap when the removal member is coupled to the cap.

Preferably, each of said at least two hooking elements comprises a cylindrical surface portion adjacent to the conical surface portion. The aforementioned cylindrical surface portions laterally abut against the cylindrical surface portion of the cap when the removal member is coupled to the cap.

Preferably, each of said at least two hooking elements comprises an abutment surface adjacent, and substantially orthogonal, to the cylindrical surface portion. This abutment surface abuts against the undercut surface portion of the cap.

The surface portions of the removal member described above allow for the aforementioned snap-fit coupling between cap and removal member, ensuring the mutual centring of these components and preventing their subsequent decoupling.

Preferably, the gripping portion is ring-shaped. This enables an effective and secure grip by the user.

The size of the ring is defined on the basis of the sizes of the average finger of different types of users (men, women, boys, etc.), so that it can be easily gripped by any kind of user.

Further characteristics and advantages of the present invention will become clearer from the following detailed description of preferred embodiments thereof, made with reference to the appended drawings and provided by way of indicative and non-limiting example. In such drawings:
- Figure 1 is a block diagram of a subcutaneous medicament dispensing device comprising a medicament dispensing assembly according to the present invention;
- Figure 2 is a perspective view of the dispensing assembly of Figure 1;
- Figure 3 is an exploded perspective view of a cartridge used in the dispensing assembly of Figure 2;
- Figure 4 is an exploded perspective view of some components of the cartridge of Figure 3;
- Figure 5 is a cross-sectional side view of a portion of the cartridge of Figure 3 in its ready to use configuration in the dispensing assembly of Figure 2;
- Figure 6 is an exploded perspective view of a perforating needle supporting member and of an injection needle supporting member of the dispensing assembly of Figure 2;
- Figure 7 is a perspective view of a component of the perforating needle supporting member of Figure 6;
- Figures 8-12 are cross-sectional side views in time sequence of a portion of the dispensing assembly of Figure 2;
- Figure 13 is a perspective sectional view of a portion of the dispensing assembly of Figure 2 at an injection needle;
- Figure 14 is a perspective sectional view of the portion of Figure 13 and of a removal member for removing a cap arranged around the injection needle;
- Figure 15 is a perspective view of the removal member of Figure 14.

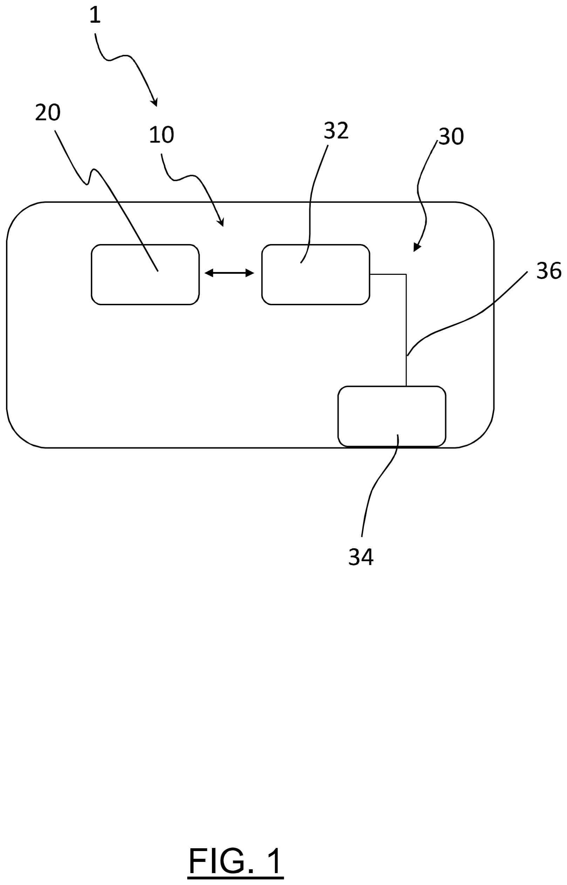

In figure 1, numeral reference 1 indicates a block diagram of a subcutaneous dispensing device for dispensing a medicament in accordance with the present invention. Preferably, the device 1 is a subcutaneous dispensing device of the wearable type.

The device 1 comprises a medicament dispensing assembly 10 also in accordance with the present invention.

The dispensing assembly 10 comprises a cartridge 20 and a fluidic path 30.

The cartridge 20 comprises a container 22 having a substantially cylindrical shape and extending along a longitudinal axis A. A medicament to be dispensed to the patient is inside the container 22. Preferably, the container 22 is made of plastic or glass.

In this description and in the subsequent claims, the terms "radially inward" and "radially outward" are used to indicate proximal and distal positions, respectively, with respect to the longitudinal axis A.

The container 22 comprises a main body 22b and, at a dispensing end portion 22a thereof, a neck 22c.

A plunger 21 can slide in a sealing manner inside the main body 22b to push the medicament towards the neck 22c.

A perforable septum 24 is provided at the dispensing end portion 22a of the container 22. The perforable septum 24 seals the container 22 on the side opposite the plunger 21.

The perforable septum 24 comprises a first portion 24a inserted into the neck 22c and a second portion 24b arranged outside the container 22.

The fluidic path 30 is configured to be travelled by the medicament when the medicament is dispensed, allowing the passage of the medicament from the cartridge 20 to the patient's body.

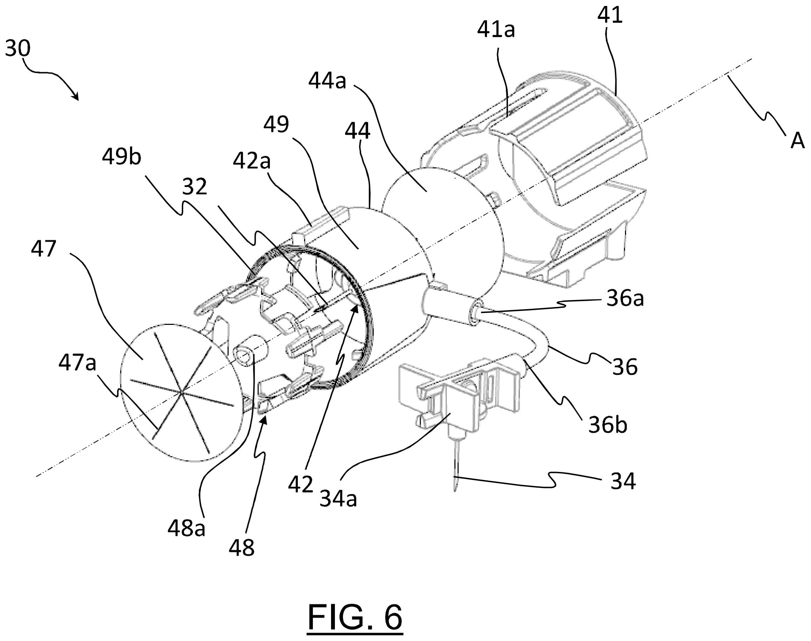

As shown for example in Figure 6, the fluidic path 30 comprises a perforating needle 32 configured to enter the container 22 at the dispensing end portion 22a by perforating the perforable septum 24, an injection needle 34 configured to be inserted into the patient's body and inject the medicament, and a flexible tube 36 that put in fluid communication the perforating needle 32 and the injection needle 34.

In particular, the flexible tube 36 has an end portion 36a connected to a needle supporting member 42 on which the perforating needle 32 is mounted and an opposing end portion 36b connected to a supporting member 34a on which the injection needle 34 is mounted.

The needle supporting member 42 and the supporting member 34a are preferably made of injection-molded plastic material and are preferably overmolded to the respective needles.

The connections of the flexible tube 36 with the needle supporting member 42 and the supporting member 34a may be made by gluing and sealing, mechanical interference or co-moulding with the plastic material of the needle supporting member 42 and the supporting member 34a.

The device 1 comprises a fluidic path opening system 30 configured to activate the perforation of the perforable septum 24 by the perforating needle 32 and thereby put the container 22 in fluid communication with the injection needle 34 through the perforating needle 32 and the flexible tube 36.

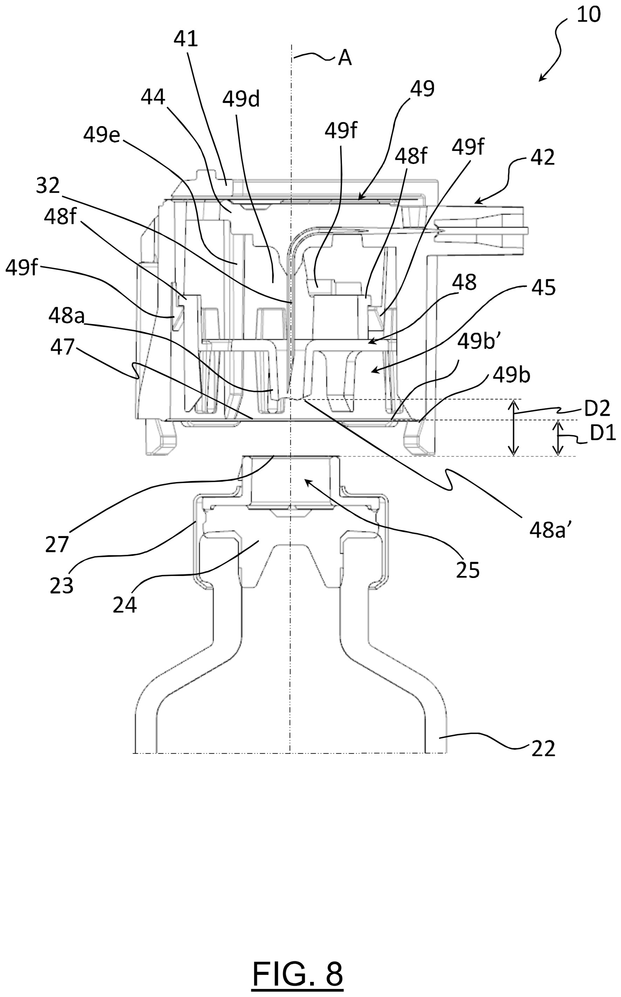

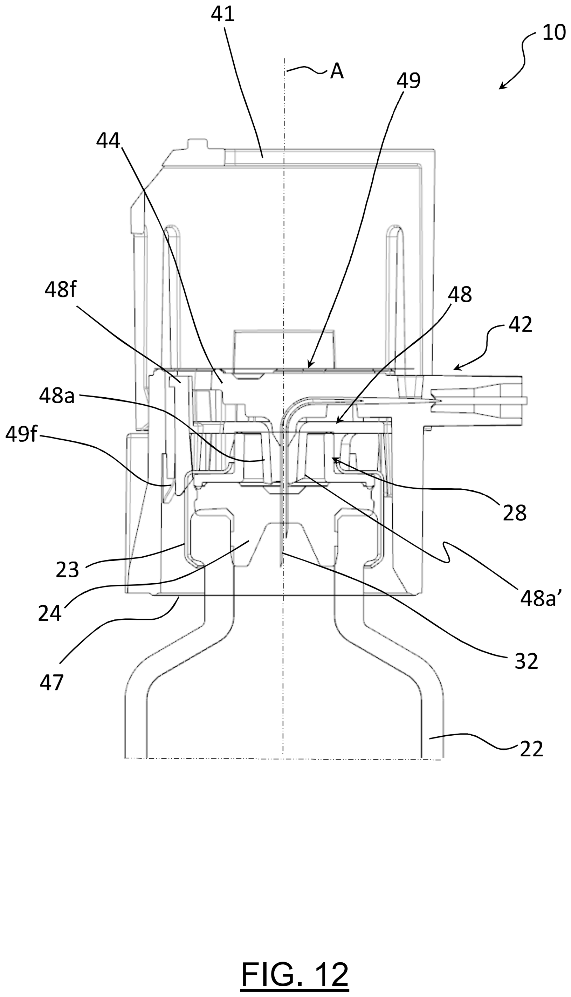

In particular, the fluidic path opening system 30 activates the translation of the needle supporting member 42 towards the cartridge 22 along the longitudinal axis A and, consequently, the passage of the dispensing assembly 10 from a first operating configuration in which the perforating needle 32 is distant from the perforable septum 24 and the fluidic path 30 is closed (Figure 8) to a second operating configuration in which the perforating needle 32 has perforated the perforable septum 24 and the fluidic path 30 is open (Figure 12).

When the dispensing assembly 10 is in the second operating configuration, the needle supporting member 42 is arranged in a radially outer position with respect to the perforable septum 24.

The dispensing assembly 10 comprises a first aseptic chamber 25 and a second aseptic chamber 45 adapted to maintain the sterility of the perforable septum 24 and perforating needle 32, respectively, until just before the perforation of the perforable septum 24 by the perforating needle 32 (Figure 8).

The first aseptic chamber 25 is defined between the perforable septum 24 and a first perforable membrane 27 associated with the container 22 at an end thereof proximal to the needle supporting member 42.

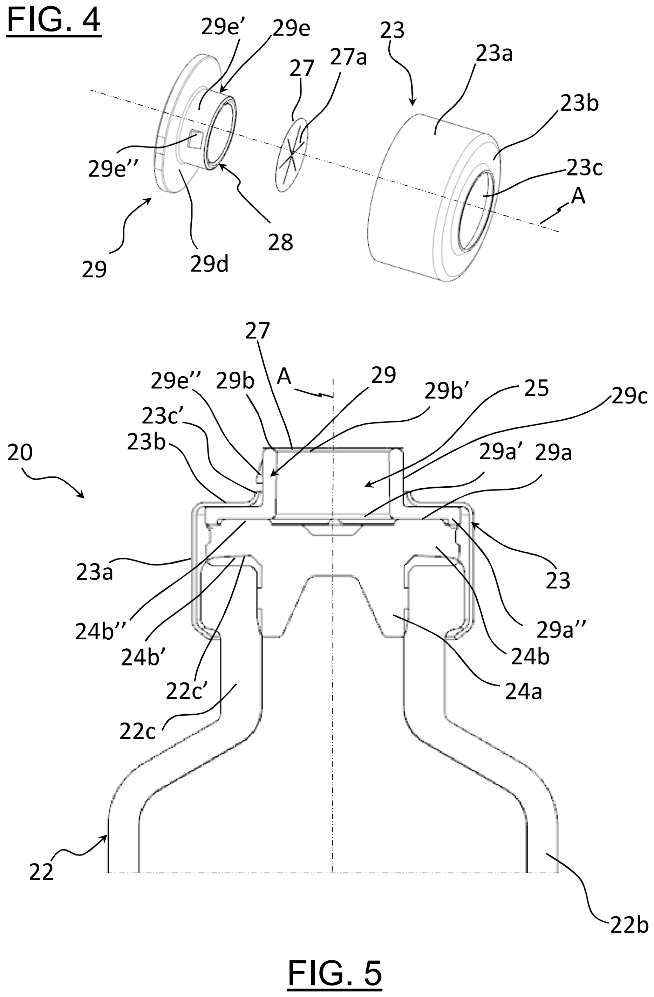

Optionally, the first perforable membrane 27 may comprise a plurality of weakening lines 27a (Figure 4). In the illustrated non-limiting example, the weakening lines 27a are arranged in a radial pattern. They may be engravings or pre-cuts made for example mechanically, chemically or by laser engraving. The weakening lines 27a are made on a surface of the first perforable membrane 27 facing the needle supporting member 42.

The second aseptic chamber 45 is defined in the needle supporting member 42 around the perforating needle 32. In particular, the second aseptic chamber 45 is defined between a closed wall 44 of the needle supporting member 42 that is distal from the cartridge 20 and a second perforable membrane 47 that is associated with the needle supporting member 42 at an end thereof proximal to the cartridge 20.

The second perforable membrane 47 comprises a plurality of weakening lines 47a, preferably arranged in a radial pattern (Figure 6). They may be engravings or pre-cuts made for example mechanically, chemically or by laser engraving. The weakening lines 47a are made on a surface of the second perforable membrane 47 facing the cartridge 20.

Preferably, the second perforable membrane 47 is totally identical, except in size, to the first perforable membrane 27.

Optionally, as in the non-limiting preferred embodiment shown in the figures, the closed wall 44 comprises an opening closed by an additional membrane 44a (Figure 6). This additional membrane 44a may be identical to the second perforable membrane 47. In the non-limiting preferred example shown, the additional membrane 44a is devoid of weakening lines.

The first perforable membrane 27, the second perforable membrane 47 and the further perforable membrane 44a can be sealed to the cartridge 20, the needle supporting member 42 and the closed wall 44, respectively, by heat welding, ultrasonic welding or bonding.

The first perforable membrane 27, the second perforable membrane 47 and the further membrane 44a may be made of aluminium, preferably coated with a polymer paint to allow heat welding to the cartridge 20, the needle supporting member 42 and the closed wall 44, respectively. In this case, a post-sterilisation treatment using radiation, e.g. gamma radiation, can be carried out. This post-sterilisation treatment makes it easy to achieve the desired sterilisation level without requiring the permeation of any gas within the fluidic path 30, thus avoiding any pressure variation within the fluidic path 30 during the post-sterilisation treatment. This avoids undesirable stresses on the seals between the perforable membranes 27, 47, 44a and the cartridge 20, the needle supporting member 42 and the closed wall 44, respectively.

Alternatively, the first perforable membrane 27, the second perforable membrane 47 and the additional membrane 44a can be made of a permeable material (e.g. medical paper or Tyvek^{™}), to allow a post-sterilisation treatment using a sterilisation gas, e.g. ethylene oxide or vaporised hydrogen peroxide (VHP).

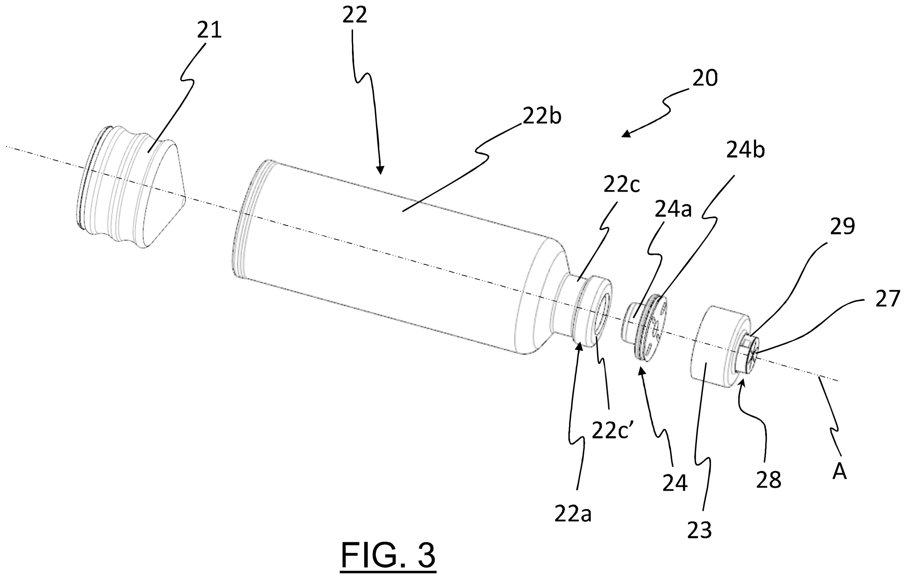

With reference to Figures 3-5, the first aseptic chamber 25 is defined within a first bushing 29 arranged coaxially to the longitudinal axis A and coupled to the perforable septum 24.

The first bushing 29 is preferably made of injection-molded plastic material.

The first bushing 29 is substantially cylindrical in shape and comprises a first annular wall 29a proximal to the container 22 and abutting against the perforable septum 24, a second annular wall 29b distal from the container 22 and thus from the perforable septum 24, and a lateral wall 29c joining the first annular wall 29a to the second annular wall 29b.

As shown in Figure 4, the aforementioned first annular wall 29a is defined in an annular flange 29d having an enlarged outer diameter.

The aforementioned second annular wall 29b is defined in an annular portion 29e having a reduced outer diameter and protruding from the annular flange 29d.

The first annular wall 29a comprises a first opening 29a' which, when the first bushing 29 is associated with the container 22, is closed by the perforable septum 24.

The second annular wall 29b comprises a second opening 29b' closed by the first perforable membrane 27.

The second annular wall 29b defines a first perforating member 28 configured to perforate the second perforable membrane 47.

The first annular wall 29a is held in direct abutment against the perforable septum 24 by a crimping ring 23 coupled to the dispensing end portion 22a of the container 22 and in direct abutment against the first bushing 29.

The annular portion 29e comprises, on a lateral surfaces 29e' thereof, an overhanging hooking element 29e".

As shown in Figure 5, the second portion 24b of the perforable septum 24 comprises a first surface 24b' abutting against a free end 22c' of the neck 22c and a second surface 24b" abutting against the first annular wall 29a of the first bushing 29.

The first annular wall 29a comprises an outer annular rim 29a" protruding towards the perforable septum 24. The perforable septum 24 is compressed by the first bushing 29 at the outer annular edge 29a".

In a variant not shown, the first annular wall 29a may comprise an additional outer annular rim projecting towards the perforable septum 24 to achieve a better or more uniform compression of the perforable septum 24.

The crimping ring 23 is preferably made of aluminium.

The crimping ring 23 is fitted onto the annular portion 29e and is in abutment against the annular flange 29d.

As shown in Figures 4 and 5, the crimping ring 23 comprises a main annular portion 23a having a predetermined diameter and a top portion 23b having a through opening 23c with a diameter smaller than said predetermined diameter. The through opening 23c is delimited by an edge 23c'. The annular portion 29e of the first bushing 29 passes through this through opening 23c.

The crimping ring 23 further comprises a locking element which, in the non-limiting example shown in Figures 4 and 5, is defined by an edge top surface 23c' and which cooperates with the hooking element 29e" to prevent the first bushing 29 from slipping out of the crimping ring 23 after the crimping ring 23 has been fitted onto the annular portion 29e of the first bushing 29 and before the crimping ring 23 is associated with the container 22.

Figures 8-12 show, in time sequence, the translational movement of the needle supporting member 42 towards the cartridge 20 in the direction of the longitudinal axis A.

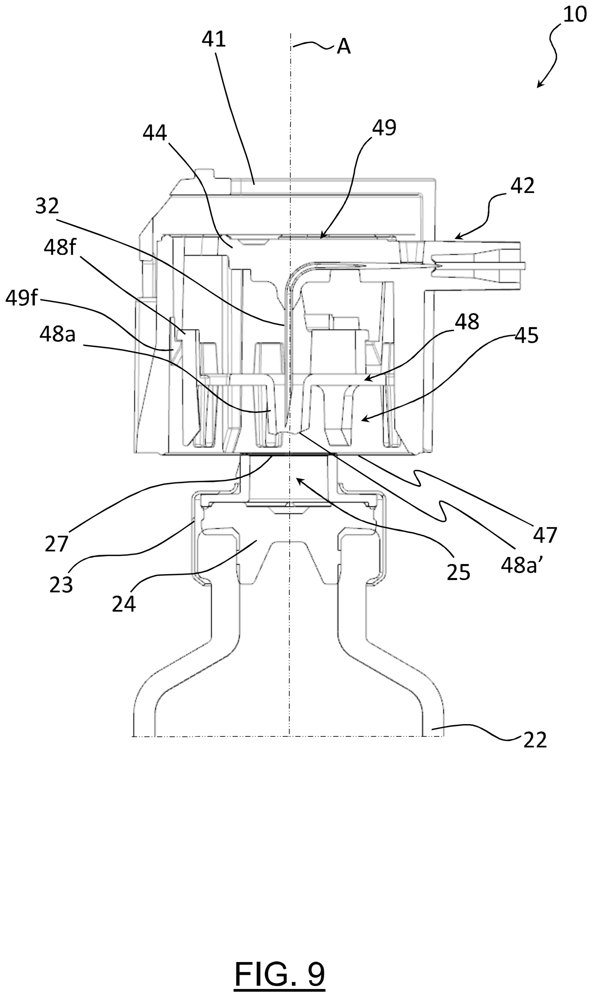

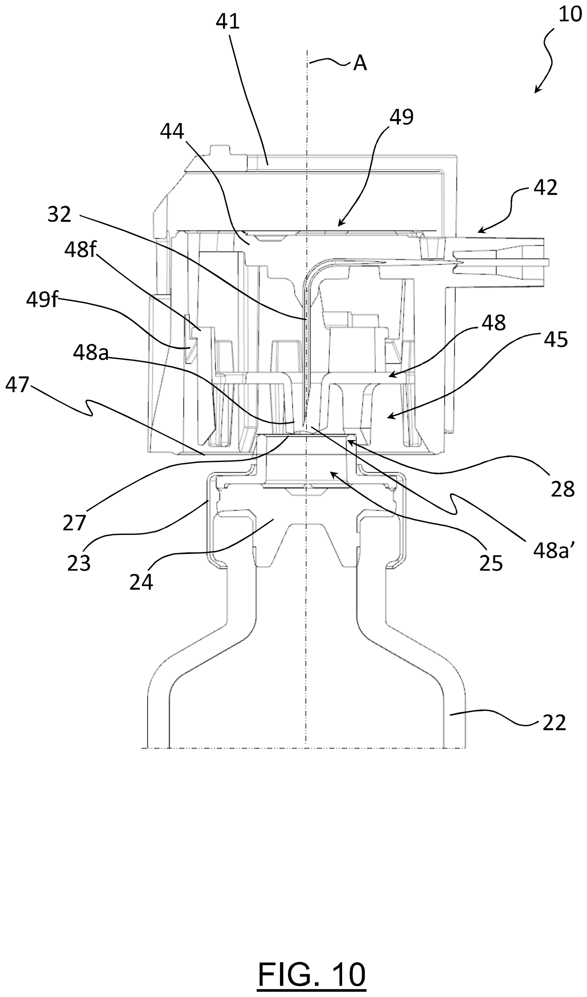

As shown in Figures 9 and 10, during a first part of the passage of the dispensing assembly 10 from its first operating configuration to its second operating configuration and before the perforating needle 32 has perforated the perforable septum 24 (Figure 12), the second perforable membrane 47 contacts the second annular wall 29b of the first bushing 29 and is perforated by the latter.

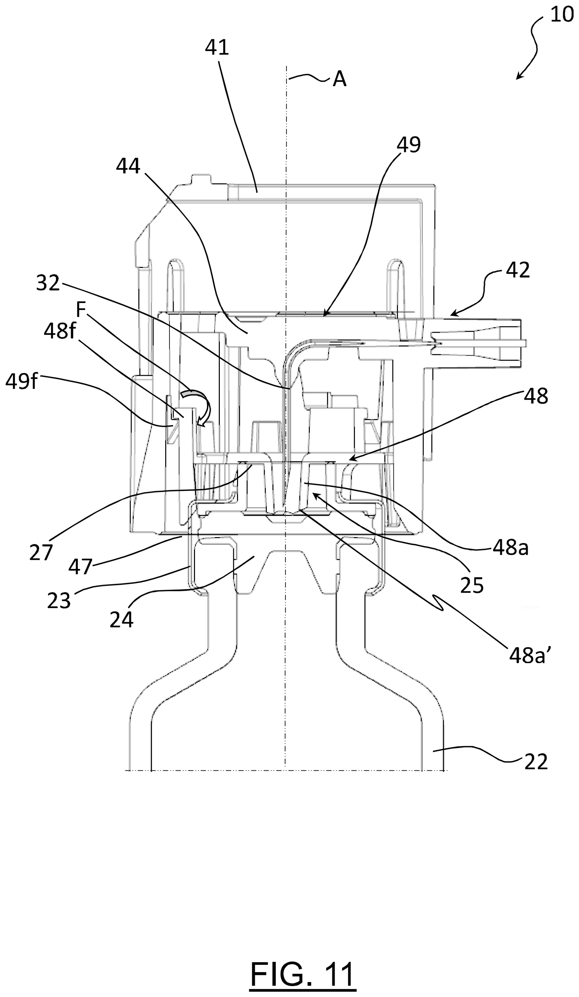

As shown in Figures 10 and 11, still during the aforementioned first part of the passage of the dispensing assembly 10 from its first operating configuration to its second operating configuration, after the second perforable membrane 37 has been perforated and still before the perforating needle 32 has perforated the perforable septum 24, the second perforating member 48 provided in the needle supporting member 42 perforates the first perforable membrane 27.

Next, the perforating needle 32 perforates the perforable septum 24, as shown in Figure 12.

As shown in Figure 8, when the dispensing assembly 10 is in its first operating configuration, the second annular wall 29b of the first bushing 29 is arranged at a first distance D1 from the second perforable membrane 47.

The second perforating member 48 is preferably made of injection-molded plastic material.

The second perforating member 48 is arranged within the needle supporting member 42. The latter is arranged within a supporting body 41 which is fixed inside the device 1.

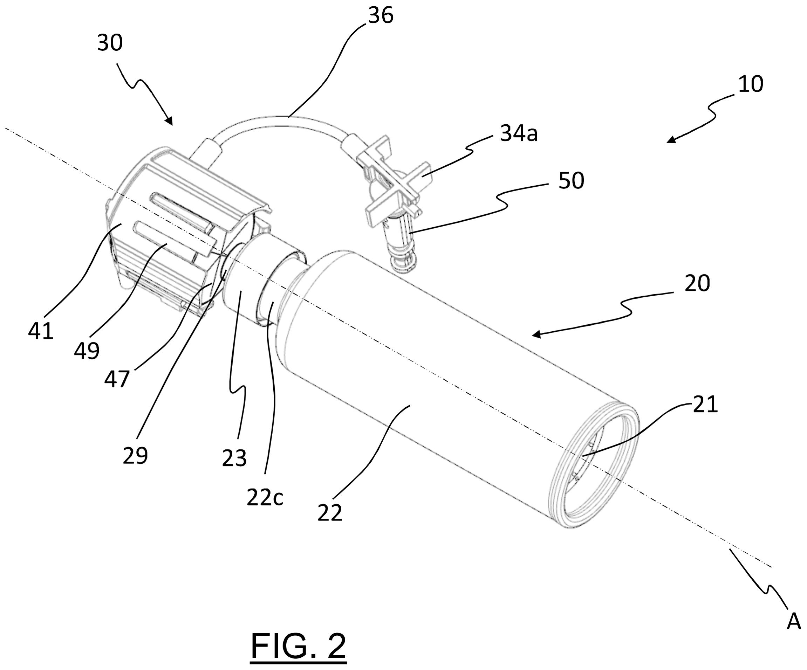

In the preferred embodiment illustrated, the support body 41 is substantially cylindrical in shape (Figures 2 and 6) and has axial grooves 41a which accommodate respective axial ribs 42a provided in the needle supporting member 42. The supporting body 41 counteracts the transverse forces generated during the movement of the needle supporting member 42 and the perforation of the first perforable membrane 27, the second perforable membrane 47 and the perforable septum 24.

In a variant not shown, the support body 41 is not present. In such a case, the needle supporting member 42 is directly mounted on an inner wall of the device 1 and is coupled to guide elements formed on that inner wall.

During the passage from the first operating configuration to the second operating configuration of the dispensing assembly 10 the needle supporting member 42 translates towards the cartridge 20 integrally with the second perforating member 48 and with respect to the support body 41, as shown in Figures 8-11.

The movement of the needle supporting member 42 towards the cartridge 20 continues until the second perforating member 48 abuts against the cartridge 20. The needle supporting member 42 continues to move towards the cartridge 20 until it reaches a stop position, as shown in Figure 12.

Referring to Figures 6-8, the needle supporting member 42 comprises a second bushing 49 having a substantially cylindrical shape.

The second bushing 49 comprises an annular wall 49b proximal to the cartridge 20, the aforementioned closed wall 44 and a side wall 49c joining the annular wall 49b to the closed wall 44.

The second bushing 49 is arranged coaxially to a longitudinal axis of the needle supporting member 42 which coincides with a longitudinal axis of the perforating needle 32. The latter coincides with the longitudinal axis A.

The second bushing 49 is preferably made of injection-molded plastic material.

The annular wall 49b comprises a respective opening 49b' closed by the second perforable membrane 47.

The second aseptic chamber 45 is defined within the second bushing 49 and the second perforating member 48 is arranged in the second aseptic chamber 45 around the perforating needle 32.

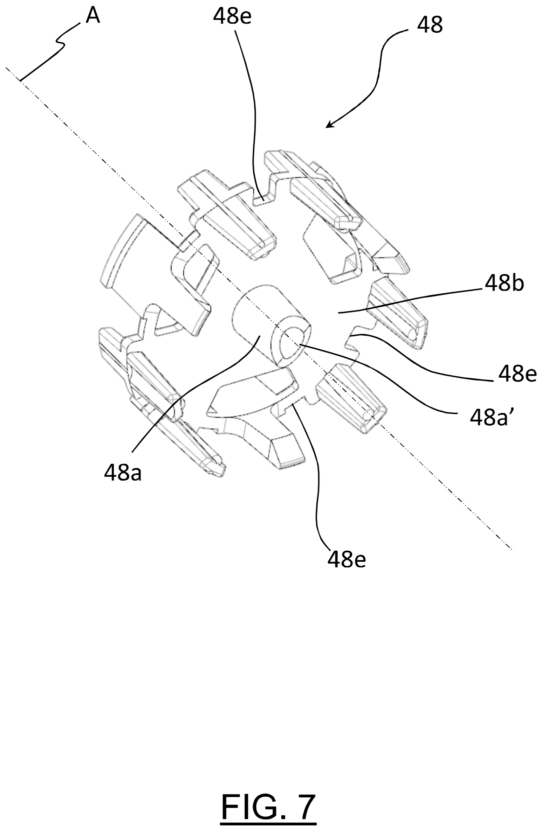

Referring to Figures 7 and 8, the second perforating member 48 comprises a central annular element 48a having a substantially cylindrical shape and arranged coaxial to the longitudinal axis A. The central annular element 48a has an end 48a' proximal to the cartridge 20 configured to perforate the first perforable membrane 27.

The central annular element 48a creates a safe passage for the perforating needle 32 and protects the latter from any unwanted contact with fragments or residues of the first membrane 27, the second membrane 47 or other components that could theoretically contaminate it prior to perforating the perforable septum 24.

As shown in Figure 8, when the dispensing assembly 10 is in its first operating configuration, the end 48a' of the central annular element 48a is arranged at a second distance D2 from the first perforable membrane 27 which is greater than the aforementioned first distance D1.

The second bushing 49 comprises, on a radially inner surface 49d thereof, a plurality of first sliding elements 49e. Correspondingly, the second perforating member 48 comprises a plurality of second sliding elements 48e coupled to the first sliding elements 49e. In particular, in the non-limiting preferred embodiment shown in Figures 7 and 8, the first sliding elements 49e are axial ribs and the second sliding elements 48e are axial grooves formed on a substantially circular plate 48b from which the central annular element 48a protrudes.

The second bushing 49 further comprises, on the radially inner surface 49d thereof, a plurality of first locking elements 49f. Correspondingly, the second perforating member 48 comprises a plurality of second locking elements 48f which are coupled to the first locking elements 49f when the dispensing assembly 10 is in its first operating configuration, as shown in Figures 8-11, and which are decoupled from the first locking elements 49f after the first perforable membrane 27 and the second perforable membrane 47 have been perforated and after the second perforating member 48 is in abutment against the cartridge 20, as shown in Figure 12. Such decoupling allows the subsequent relative movement of the needle supporting member 42, and of the perforating needle 32 which is integral therewith, with respect to the second perforating member 48 and the perforation of the perforable septum 24 by the perforating needle 32.

The aforementioned decoupling occurs as a result of a deformation of the second perforating member 48 when the second perforating member 48 continues to be pushed against the cartridge 22 after it has abutted against the cartridge 20. In particular, as indicated by the arrow F in Figure 11, the decoupling between the second locking elements 48f and the first locking elements 49f occurs by as a result of a rotation of the second locking elements 48f with respect to the first locking elements 49f.

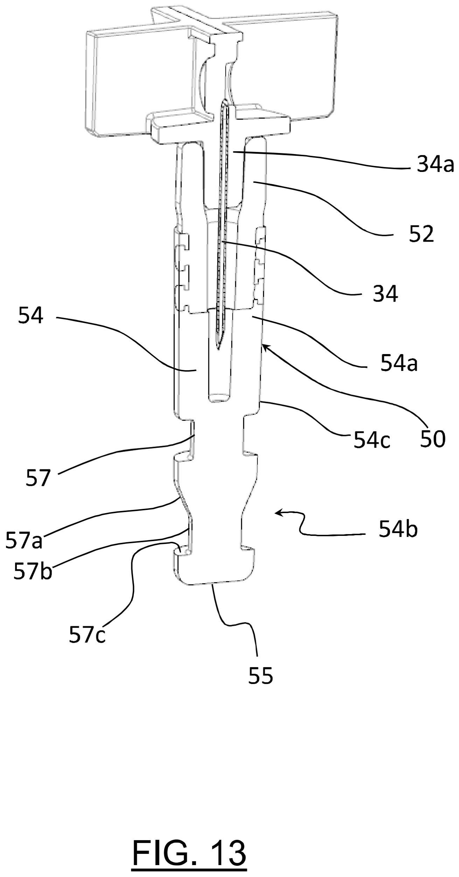

Referring to Figures 2, 13 and 14, the dispensing assembly 10 further comprises a cap 50 arranged around the injection needle 34. The purpose of this cap 50 is to protect the injection needle 34 and maintain its sterility.

The cap 50 extends along a longitudinal direction that coincides with an axial direction B of the injection needle 34.

The cap 50 comprises a first cylindrical element 52 made of a soft plastic material and configured to be coupled to a supporting member 34a of the injection needle 34, and a second cylindrical element 54 made of a rigid plastic material and co-molded to the first cylindrical element 52.

The second cylindrical element 54 comprises a first end portion 54a coupled to the first cylindrical element 52 and an opposing second end portion 54b having a closed end wall 55.

The second cylindrical element 54 comprises, on a side surface 54c thereof, a recess 57 adapted to allow the cap 50 to be held in position with respect to the device 1 during coupling between the cap 50 and a removal member 70 described below.

The side surface 54c comprises, at the second end portion 54b, a conical surface portion 57a close to the recess 57, a cylindrical surface portion 57b adjacent to the conical surface portion 57a and an undercut surface portion 57c adjacent to the cylindrical surface portion 57b.

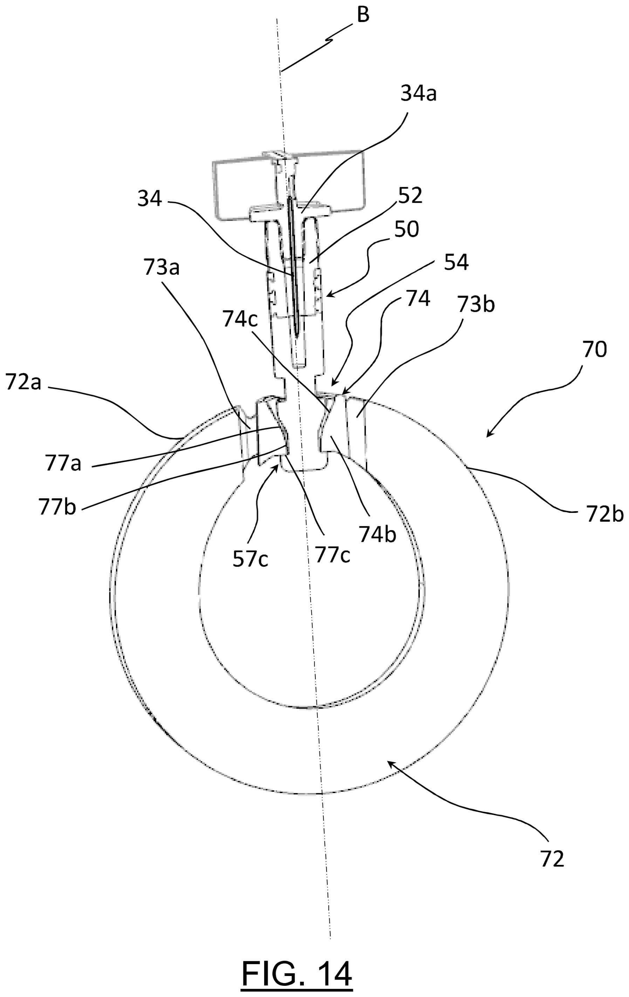

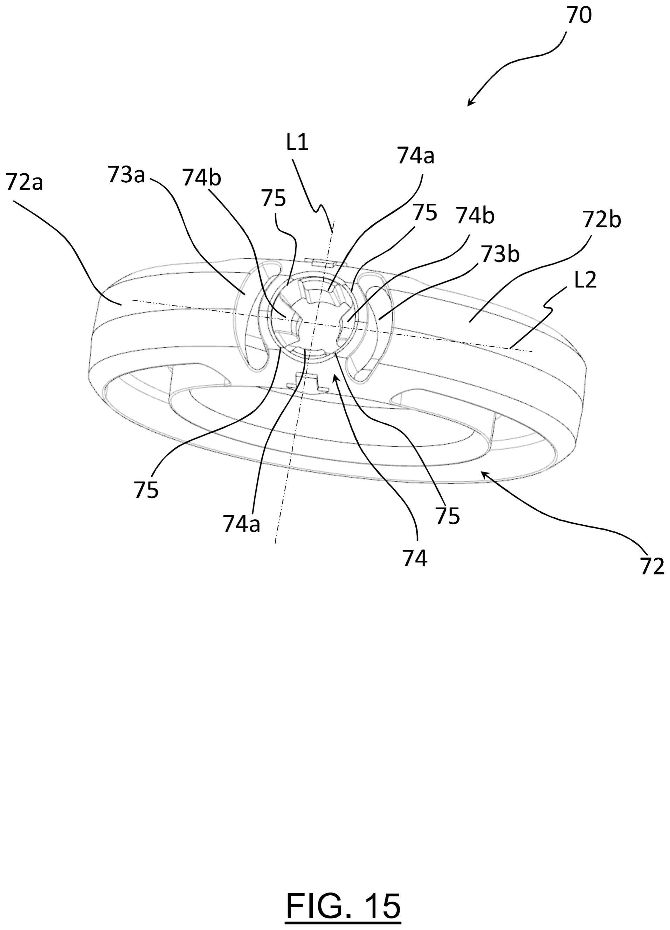

Since the shape and/or size of the cap 50 may not be suitable for allowing an easy manual removal of the cap by a user, a removal member 70 is associated with the cap (Figures 14 and 15).

The removal member 70 comprises a gripping portion 72 configured to interface with the user's fingers in order to remove the cap 50 and release the injection needle 34, and a coupling portion 74 snap-coupled to the cap 50.

The gripping portion 72 and the coupling portion 74 are made in a single piece.

In the non-limiting preferred embodiment shown in Figures 14 and 15, the gripping portion 72 is ring-shaped.

The coupling portion 72 comprises two parts 72a and 72b extending from opposing sides with respect to the coupling portion 74.

A respective through opening 73a, 73b is provided between each of the two parts 72a, 72b and the coupling portion 74.

As shown in Figure 15, the coupling portion 74 comprises two guide elements 74a facing and aligned with each other along a first direction L1. In a variant not shown, there could be more than two guide elements 74a.

The coupling portion 74 further comprises two hooking elements 74b facing and aligned with each other along a second direction L2 orthogonal to the first direction L1. In a variant not illustrated, more than two hooking elements 74b could be provided.

As shown in Figure 14, each of the two hooking elements 74b comprises a lateral surface 74c having a conical surface portion 77a, a cylindrical surface portion 77b adjacent to the conical surface portion 77a and an abutment surface 77c adjacent to, and substantially orthogonal to, the cylindrical surface portion 77b.

The conical surface portion 77a is coupled to the conical surface portion 57a of the cap 50, the cylindrical surface portion 77b is coupled to the cylindrical surface portion 57b of the cap 50, and the abutment surface 77c is coupled to the undercut surface portion 57c of the cap 50.

As shown in Figure 15, the coupling portion 74 further comprises, between each guide element 74a and each hooking element 74b, a zone 75 having a reduced thickness. The latter and the through openings 73a, 73b allow the elastic deformation of the coupling portion 74 when it is coupled to the cap 50.

In order to couple the removal member 70 to the cap 50, the gripping portion 72 is pushed against the second end portion 54b of the cap 50 along the longitudinal direction of the cap 50 until the snap-fit coupling between the removal member 70 and the cap 50 is achieved as a result of the elastic deformation of the coupling portion 74 of the removal member 70.

The snap-fit coupling of the removal device 70 to the cap 50 takes place as described below. The second cylindrical element 54 of the cap 50 enters the coupling portion 74, the two hooking elements 74b couple with interference with the second end portion 54b of the cap 50 and move along the second direction L2 due to the presence of the two through openings 73a, 73b and the zones 75 with a reduced thickness. The two hooking elements 74b return to their original position when the cylindrical surface portions 77b are located at the cylindrical surface portion 57b of the cap 50. In this condition, the abutment surface 77c of the removal member 70 abuts against the undercut portion 57c of the cap 50.

The coupling between the removal member 70 and the cap 50 is therefore irreversible. Accordingly, after the cap 50 and the removal member 70 have been coupled, they cannot be separated but only removed from the device 1 as one piece by a pulling action exerted by a user on the gripping portion 72 prior to application of the device 1 on the patient's skin.

The removal member 70 is preferably made of injection-molded plastic material and is hooked to the cap 50 in the last assembly stages of the device 1 and before its final packaging.

Obviously, a person skilled in the art, in order to meet specific requirements, may make numerous modifications to the present invention, all of which are within the scope of protection defined by the following claims.

## Claims

1. Dispensing assembly (10) for dispensing a medicament, comprising:
- a cartridge (20) comprising a container (22) containing the medicament to be dispensed and a perforable septum (24) coupled to the container at a dispensing end portion (22a) of the container (22);
- a perforating needle (32) configured to enter the container (22) at said dispensing end portion (22a) by perforating the perforable septum (24);
- an injection needle (34) configured to inject the medicament to a patient, said injection needle (34) defining with the perforating needle (32) a fluidic path (30) configured to be travelled by the medicament during the dispensing of the medicament;
- a needle supporting member (42) on which the perforating needle (32) is mounted;
- a first aseptic chamber (25) defined between the perforable septum (24) and a first perforable membrane (27) associated with the container (22) at one end thereof proximal to the needle supporting member (42);
- a second aseptic chamber (45) defined in the needle supporting member (42) around the perforating needle (32) between a closed wall (44) which is distal from the cartridge (20) and a second perforable membrane (47) associated with the needle supporting member (42) at one end thereof proximal to the cartridge (20);
wherein the dispensing assembly (10) is configured to pass from a first operating configuration in which the perforating needle (32) is distant from the perforable septum (24) and the fluidic path (30) is closed and a second operating configuration in which the perforating needle (32) has perforated the perforable septum (24) and the fluidic path (30) is open;
wherein during a first part of the passage from said first operating configuration to said second operating configuration and before the perforating needle (32) has perforated the perforable septum (24), said second perforable membrane (47) is perforated by a first perforating member (28) associated with the cartridge (20) and the first perforable membrane (27) is perforated by a second perforating member (48) associated with the needle supporting member (42), and during a second part of the passage from said first operating configuration to said second operating configuration and after said first perforable membrane (27) and second perforable membrane (47) have been perforated, the perforating needle (32) perforates the perforable septum (34).

2. Dispensing assembly (10) according to claim 1, wherein during the first part of the passage from said first operating configuration to said second operating configuration the second perforable membrane (47) is perforated by the first perforating member (28) before the first perforable membrane (27) is perforated by the second perforating member (48).

3. Dispensing assembly (10) according to claim 1 or 2, wherein the passage from said first operating configuration to said second operating configuration occurs as a result of a translational movement of the needle supporting member (42) towards the cartridge (20) and wherein:
- in said first part of the passage from said first operating configuration to said second operating configuration, the needle supporting member (42) has an initial configuration in which the second perforating member (48) moves towards the cartridge (20) integrally with the needle supporting member (42);
- in said second part of the passage from said first operating configuration to said second operating configuration, the needle supporting member (42) has a final configuration in which the second perforating member (48) is in abutment against the cartridge (20) and the needle supporting member (42) moves towards the cartridge (20).

4. Dispensing assembly (10) according to any one of the previous claims, wherein the first aseptic chamber (25) is defined within a first bushing (29) coupled to the perforable septum (25) and having:
- a first annular wall (29a) in abutment against the perforable septum (24) and comprising a first opening (29a') closed by the perforable septum (24);
- a second annular wall (29b) distal from the perforable septum and comprising a second opening (29b') closed by the first perforable membrane (27);
- a side wall (29c) joining the first annular wall (29a) to the second annular wall (29b).

5. Dispensing assembly (10) according to claim 4, wherein the first perforating member (28) is defined by said second annular wall (29b).

6. Dispensing assembly (10) according to any one of the previous claims, wherein the needle supporting member (42) comprises a second bushing (49) having:
- an annular wall (49b) proximal to the cartridge (20) and comprising a respective opening (49b') closed by the second perforable membrane (47);
- said closed wall (44) distal from the cartridge (20);
- a side wall (49c) joining the annular wall (49b) proximal to the cartridge (20) to the closed wall (44) distal from the cartridge (20); wherein the second aseptic chamber (45) is defined within the second bushing (49) and the second perforating member (48) is arranged in said second aseptic chamber (45) around the perforating needle (32).

7. Dispensing assembly (10) according to any one of the previous claims, wherein the second perforating member (48) comprises a central annular element (48a) surrounding the perforating needle (32) and having an end (48a') proximal to the cartridge (20) and configured to perforate the first perforable membrane (27) during said first part of the passage from said first operating configuration to said second operating configuration.

8. Dispensing assembly (10) according to claim 7 when depending on claim 5, wherein when the dispensing assembly (10) is in its first operating configuration the second annular wall (29b) of the first bushing (29) is arranged at a first distance (D1) from the second perforable membrane (47) and the end (48a') proximal to the cartridge (20) of said central annular element (48a) is arranged at a second distance (D2) from the first perforable membrane (27), wherein the first distance (D1) is less than the second distance (D2).

9. Dispensing assembly (10) according to any one of claims 6 to 8, wherein the second bushing (49) comprises, on a radially inner surface thereof (49d), at least one first sliding element (49e) and at least one first locking element (49f), and wherein the second perforating member (48) comprises at least one second sliding element (48e) coupled to the at least one first sliding element (49e) and at least one second locking element (48f) configured to be coupled to the at least one first locking element (49f) when the dispensing assembly (10) is in its first operating configuration and to decouple from the at least one first locking element (49f) after said first perforable membrane (27) and second perforable membrane (47) have been perforated and after the second perforating member (48) is in abutment against the cartridge (20), wherein the second perforating member (48) is made of a material such that, following the abutment between the second perforating member (48) and the cartridge (20), the second perforating member (48) is deformed so as to decouple the at least one second locking element (48f) from said at least one first locking element (49f).

10. Dispensing assembly (10) according to any one of the previous claims, wherein the second perforable membrane (47) comprises a plurality of weakening lines (47a) arranged in a radial pattern.

11. Subcutaneous dispensing device (1) for dispensing a medicament comprising a dispensing assembly (10) according to any one of the previous claims.

12. Method for making an aseptic connection between a cartridge (20) containing a medicament to be dispensed and a perforating needle (32), wherein the cartridge (20) comprises a container (22) containing the medicament to be dispensed, a perforable septum (24) coupled to the container (20) and a first aseptic chamber (25) defined between the perforable septum (24) and a first perforable membrane (27) associated with the container (22) at an end thereof proximal to the perforating needle (32), and wherein the perforating needle (32) is arranged within a second aseptic chamber (45) defined in a needle supporting member (42) between a closed wall (44) distal from the cartridge (20) and a second perforable membrane (47) associated with the needle supporting member (42) at an end thereof proximal to the cartridge (20), the method comprising:
- perforating the second perforable membrane (47) by a first perforating member (28) associated with the cartridge (20), and the first perforable membrane (27) by a second perforating member (48) associated with the needle supporting member (42) and, subsequently,
- perforating the perforable septum (24) by the perforating needle (32).

13. Method according to claim 12, wherein the second perforable membrane (47) is perforated by the first perforating member (28) before the first perforable membrane (27) is perforated by the second perforating member (48).

14. Method according to claim 12 or 13, wherein the perforation of the first perforable membrane (27) and the second perforable membrane (47) occurs as a result of a translational movement of the needle supporting member (42) towards the cartridge (20) and wherein the perforation of the perforable septum (24) occurs after the second perforating member (48) has abutted against the cartridge (20) as a result of a translational movement of the needle supporting member (42) towards the cartridge (20) and with respect to the second perforating member (48).
